# EUROPEAN PATENT SPECIFICATION

(11) **EP 1 036 810 B1**
(45) Date of publication and mention of the grant of the patent: **01.03.2006**
(21) Application number: 99310586.5
(22) Date of filing: 24.12.1999
(51) Int. Cl.: C08J 5/02, C08J 7/04, A41D 19/00, C08L 21/00

(54) **Elastomeric article**
Elastomerischer Gegenstand
Objet élastomère

(30) Priority: 13.03.1999 MY 9900939
(43) Date of publication of application: 20.09.2000
(73) Proprietor: Ansell Medical SDN.BHD., Melaka 75450 (MY)
(72) Inventor: Lai, Hee-Meng, 75350 Melaka (MY); Cacioli, Paul, 75350 Melaka (MY); Kwan, Soo-Hwa, Cheng, 75250 Melaka (MY); Ng, Soon-Kiang, Beruang, 75350 Melaka (MY); Kassim, Adeli, 75450 Melaka (MY); Nasaruddin, Wan, Durong, 75450 Melaka (MY); Husin, Yunus, Air Molek, 75460 Melaka (MY); Tan, Yong-Poon, 75150 Melaka (MY)
(74) Representative: Knowles, James Atherton

(56) References cited:
- EP-A- 0 640 623
- EP-A- 0 681 912
- EP-A- 0 714 961
- EP-A- 0 906 731
- GB-A- 2 292 384
- US-A- 4 143 109

## Description

### BACKGROUND OF THE INVENTION

The present invention relates to elastomeric articles which exhibit enhanced lubricity with respect to both dry and damp surfaces and in particular to flexible elastomeric articles such as films and gloves which are powder free and exhibit enhanced lubricity with respect to both dry and damp surfaces such as skin.

Elastomeric articles such as medical gloves are generally required to be tight fitting. This requirement makes donning the gloves difficult unless they are lubricated. It has been common practice to utilise powder lubricants such as modified com starch or talc applied to the inner surface of medical gloves to facilitate donning. However, certain post-operative complications including adhesions, peritonitis and granuloma formation have been attributed to the use of loose powder as a lubricant on gloves and other items used in surgery.

Much effort has been devoted to developing medical gloves which may be readily donned but which do not utilise a powder lubricant. Approaches which have been tried include depositing a granular material on the inner surface of the gloves, bonding to the outer elastomeric layer an inner layer comprising embedded particles, halogenating the gloves and bonding a lubricating hydrogel polymer to the inner surface of the glove and treating the surface with a surfactant.

EP-A-0640623 relates to rubber articles produced by latex dipping, and in particular, to a method for the production of powder free natural rubber latex medical gloves.

EP-A-0714961 relates to compositions based on polyethylenic waxes and polymers derived from dimethylsiloxane for coating, with a lubricating layer, flexible rubber articles and, in particular, fine-film latex gloves.

US-A-4143109 relates to a method of making a medical glove to be donned without the use of additional lubricants including applying a layer on to a glove form, said layer being a particulate suspension.

Although each of the above approaches has its merits, none provide a medical glove which may be as readily donned as a powdered glove, especially when the hands are damp or wet.

We have now found an elastomeric article comprising a coating which provides the article with enhanced lubricity with respect to both dry and wet or damp surfaces.

### SUMMARY OF THE INVENTION

Accordingly, the present invention provides an elastomeric article having a layer of natural or synthetic rubber to which is applied at least one layer of a coating of a polymer blend characterised in that the polymer blend includes at least one film forming polymer and at least one wax.

In another aspect, the present invention also provides a process for making an elastomeric article comprising forming a first layer of natural or synthetic rubber followed by coating at least one surface of the first layer with a layer of a polymer blend comprising a film forming polymer and a wax.

### DETAILED DESCRIPTION OF THE INVENTION

Preferred elastomeric articles according to the invention are medical gloves and the present invention is described primarily with reference to surgeons' gloves. However, the elastomeric articles of the invention may include other elastomeric articles and particularly elastomeric articles which may have contact with skin or other tissue. Such articles include: films, sheets, examination gloves used by doctors, veterinary practitioners, dentists, nurses and other personnel coming into contact with animals or animal fluids; finger stalls; condoms; catheters, ureters, and sheath type incontinence devices.

Suitable elastomers which may be used for the first or rubber layer of the elastomeric articles of the present invention include natural rubber latex, nitrile rubber latex, polychloroprene, styrene-butadiene rubber or a polyurethane. Preferably the first or rubber layer of the elastomeric article comprises natural latex rubber and more preferably, the first or rubber layer of the elastomeric article comprises natural latex rubber glove.

In the elastomeric article of the present invention, the film forming polymer component of the polymer blend coating may be selected from a range of film forming polymers including acrylic polymers and copolymers, methacrylate polymers and copolymers, polyurethane polymers and carboxylated styrene-butadiene copolymers.

Preferably the film forming polymer of the polymer blend is a polyurethane. Suitable polyurethanes include aliphatic polyurethanes and aromatic polyurethanes. Preferably the polyurethane is used in the form of an aqueous dispersion. Examples of suitable commercially available polyurethanes include, but are not limited to, BEETAFIN™ L9009, Witcobond™ W-506, and Neorez™ R-972.

In the elastomeric article of the present invention, the wax component is preferably a synthetic polymer or copolymer wax and more preferably a polyethylene wax, high density polyethylene wax, an oxidised or modified polyethylene wax or high density polyethylene wax or a mixture of such waxes. Preferably, the wax has a melting point equal to or above 80°C and more preferably the wax is a polyethylene wax having a melting point equal to or above 80°C. Examples of suitable commercially available polyethylene waxes include, but are not limited to, AQUAMAT™ 213, MICHEM LUBE™ 61335 and AQUACER™ 502.

The polymer blend coating used in the present invention may also comprise conventional additives and modifiers including surfactants, antioxidants, antistatic agents, bactericidal or bacteriostatic agents and hardness modifiers. Suitable bactericidal and/or bacteriostatic agents may be selected from the known group of quaternary amine compounds. Suitable hardness modifiers may be selected from polymeric compounds including, but not limited to, poly(2-hydroxyethylmethacrylate), acrylic polymers and melamine-formaldehyde resins. Preferably, the hardness modifier is a polymer or copolymer having a glass transition temperature (Tg) higher than that of the film forming polymer.

In the elastomeric article of the present invention, the ratio of film forming polymer to wax in the polymer blend is preferably at least 0.5 to 1 and more preferably between 0.5 to 1 and 20 to 1.

Preferably the polymer blend is applied to the first layer of natural or synthetic rubber in the form of an aqueous dispersion or emulsion. For example, the film forming polymer and the wax are dispersed in water in the presence of one or more suitable surfactants. Suitable surfactants may be chosen from a range of commercially available non-ionic and anionic surfactants including for example, those sold under the trade marks DARVAN™ and SYNPERONIC™. Typically the aqueous dispersion of the polymer blend comprises 3 to 30% by weight solids and more preferably 5 to 20% by weight solids. The concentration of the surfactant(s) is preferably 0.01 to 0.10% (w/v), most preferably 0.02 to 0.05% (w/v).

The elastomeric articles of the present invention may be made by forming the first layer of natural or synthetic rubber and then applying to at least one surface of the first layer, a coating of a polymer blend comprising at least one film forming polymer and at least one wax.

Between formation of the first layer and coating with the polymer blend, the first layer of natural or synthetic rubber optionally may be heated until the layer is gelled or the surface of the first layer to be coated may be leached.

Optionally the surface of the first layer which is to be coated with the polymer blend may be treated to improve adhesion between the first layer and the polymer blend. Suitable methods for surface treatment of the first layer may include treatment with an oxidising agent such as an aqueous solution of an alkali metal or alkaline earth metal hypochlorite followed by leaching in water, preferably hot water. Alternatively, the surface of the first layer which is to be coated with the polymer blend may be treated with an agent, such as a dilute aqueous solution of aluminium sulfate or calcium nitrate, to promote deposition of the polymer blend onto the surface of the first layer.

For convenience, the process of the present invention may be illustrated by reference to surgeons' gloves. In such a process a conventional method for preparing latex rubber gloves, such as described in a bulletin "Dipping With Natural Rubber Latex" by the Malaysian Rubber Producers' Research Association, Hertford, England, 1980, is employed. For example, a former coated with a coagulant is first dipped into natural or synthetic latex. , After withdrawal, the glove on the former is heated until the film is significantly gelled. The glove may then be subjected to leaching.

The polymer blend may then be applied to the first layer of natural or synthetic rubber by dipping the glove on the former into a bath of an aqueous dispersion and/or solution of the polymer blend.

The first layer of natural or synthetic rubber can be 0.10-0.40 mm, preferably 0.15-0.30 mm. The layer of polymer blend is preferably 1-10 µm, most preferably 2-5 µm.

After application of the polymer blend the glove is then dried and cured. This improves adhesion of the polymer blend coating to the rubber. The curing can be conducted at a temperature which approaches, is similar to, or exceeds, the melting point of the wax used in the polymer blend, so long as it results in bonding of the polymer blend to the rubber base.

The resulting glove may be further treated according to methods known in the art to further improve glove donning and/or to reduce blocking. Such further treatment includes treatment with a silicone by, for example, contacting with an aqueous dispersion or emulsion or solution of a silicone in the presence of a surfactant.

As hereinbefore indicated, the elastomeric articles or the present invention have improved lubricity with respect to dry, wet or damp surfaces. For example, surgeons' gloves according to the present invention show excellent donning properties with respect to dry, damp or wet hands. Moreover, the elastomeric articles of the present invention may be readily manufactured and show enhanced lubricity without the need to use conventional powder lubricants.

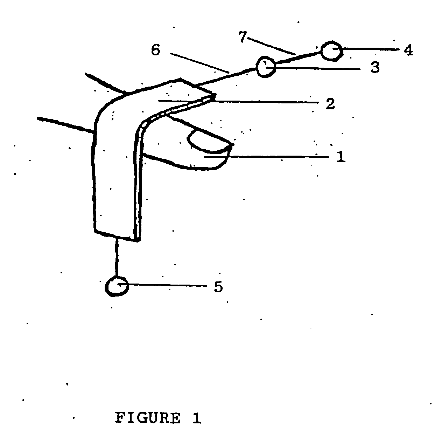

Figure 1 is a diagrammatic representation of the measurement of lubricity of an elastomeric article.

In the measurement of lubricity, a strip of the elastomeric article 15 mm x 150 mm (2) is placed between the first and second knuckle of the fore-finger (1) such that the surface to be measured is in contact with the finger. The leading section of the test piece (2) is connected to a load cell (3) which is mounted on a sledge linked to a constant speed driving mechanism (4). The trailing section of the test piece is attached to a three gm load (5). The finger is positioned such that the leading section of the test piece, the length of nylon filament (6), the length of the horizontal sledge and the length of nylon filament (7) are parallel. The test piece is moved around the finger at around a speed of 2.5 mm per second for a distance of approximately 50 mm and the average friction force recorded.

The results obtained utilising this test clearly show that elastomeric articles of the present invention have a significantly higher lubricity than prior art elastomeric articles.

The present invention is now illustrated in greater detail by the following specific examples. It is to be understood that these examples are given by way of illustration and are not to be interpreted as limiting the scope of the claims. All percentages in the examples, and elsewhere in the specification, are by weight unless otherwise specified.

### Example 1

A surgeons' glove of natural rubber latex was formed by conventional dipping process. Upon withdrawing from the latex bath, the glove on the former was heated until the film was sufficiently gelled. The glove was then leached in hot water, treated with 2% dilute sodium hypochlorite, leached again in hot water and dried out before dipping into a dispersion of a polymer blend, the composition of which is described in Table I.

**Table I**

| | **Parts by Weight** |
|---|---|
| Film forming polymer (Beetafin PU L9009) | 10 |
| Polyethylene wax (Aquamat 213) | 3 |
| Hardness Modifier (Cymel 373) | 1 |
| Surfactant (Darvan WAQ) | 0.025 |
| Deionized water | 86 |

After curing, the .glove was leached and treated with silicone emulsion consisting of 0.05 to 0.25% of silicone and 0.05 to 0.25% of cetyltrimethyl-ammonium chloride. The finished glove shows good dry, damp and wet donning properties and is found to have less than 2mg of powder mass/glove as measured in accordance with ASTM D 6124-97.

### Example 2

Example 1 was repeated except that the glove was dipped into a 0.5% solution of aluminium sulphate prior to coating and the glove was not treated with silicone emulsion. Application of alum allows a thicker and more uniform coating to be formed. The coating exhibits good lubricity with respect to dry, damp and wet hands.

### Example 3

Example 1 was repeated except that the glove was dipped into a 0.5% solution of aluminium sulphate prior to coating. Application of alum allows a thicker and more uniform coating to be formed. The coating exhibits good lubricity with respect to dry, damp and wet hands.

### Example 4

Example 2 was repeated except that the coating was replaced by the polymer blend indicated in Table II.

**Table II**

| | **Parts by Weight** |
|---|---|
| Film forming polymer (Witcobond W-506) | 6.2 |
| Polyethylene wax (Michem Lube 61335) | 2.0 |
| Hardness Modifier (Cymel 373) | 0.7 |
| Surfactant (Darvan WAQ) | 0.018 |
| Surfactant (Synperonic NP40) | 0.019 |
| Deionized Water | 91 |

The finished glove shows good dry, damp and wet donning properties and is found to have less than 2mg of powder mass/glove as measured in accordance with ASTM D 6124-97.

### Example 5

Example 4 was repeated except that after curing, the glove was leached and treated with silicone emulsion consisting of 0.05 to 0.25% of silicone and 0.05 to 0.25% of cetyltrimethylammonium chloride. The finished glove shows good dry damp and wet donning properties.

### Example 6

A thin-walled glove of chloroprene latex was formed by conventional dipping process. Upon withdrawal from the latex bath, the glove on the former was heated until it was sufficiently gelled. The glove was then leached in hot water before dipping into a dispersion of a polymer blend, the composition of which is given in Table III.

**Table III**

| | **Parts by Weight** |
|---|---|
| Film forming polymer (NeoRez R-972) | 10 |
| Polyethylene wax (Aquacer 502) | 3 |
| Hardness Modifier (Cymel 373) | 1 |
| Surfactant (Darvan WAQ) | 0.025 |
| Deionized water | 86 |

After curing, the glove was treated with silicone emulsion comprising 0.05 to 0.25% of silicone and 0.05 to 0.25% of cetyltrimethylammonium chloride. The finished glove shows good dry, damp and wet donning properties.

### Example 7

The skin lubricity of the elastomeric articles of the present invention was compared with the skin lubricity of prior art elastomeric articles according to the following test.

With reference to Figure 1 a strip of the elastomeric article 15 mm x 150 mm (2) was placed between the first and second knuckle of the forefinger (1) such that the surface to be measured was in contact with the finger. The leading section of the test piece (2) was connected to a load cell (3) which was mounted on a sledge linked to a constant speed driving mechanism (4). The trailing section of the test piece was attached to a 3 gm load (5). The finger was positioned such that the leading section of the test piece, the length of nylon filament (6), the length of the horizontal sledge and the length of nylon filament (7) were parallel. The test piece was moved around the finger at a speed of 2.5 mm/sec for a distance of approximately 50 mm and the average friction force recorded.

A measure of lubricity, hereinafter referred to as "Lubricity Number" was obtained by dividing the dry friction force measured for natural, uncoated, non-powdered rubber (Comparative Example 1) by the friction force measured for each sample. The results, recorded in Table IV, are the averages of nine determinations from three individuals. The higher the Lubricity Number the higher the lubricity.

**Table IV**

| **Elastomeric Article**^{**1**} | **Friction Force (g)** | **Lubricity Number** |
|---|---|---|
| Comparative Example 1² | 59.9 | 1.0 |
| Comparative Example 2³ | 6.2 | 9.7 |
| Comparative Example 3⁴ | 23.2 | 2.6 |
| Example 2 | 7.0 | 8.6 |
| Example 3 | 6.3 | 9.5 |
| Comparative Example 4⁵ | 12.7 | 4.7 |
| Example 4 | 7.6 | 7.9 |
| Example 5 | 6.3 | 9.5 |

| | | |
|---|---|---|
| 1. Samples taken from formed surgeons' gloves. | | |
| 2. Natural rubber latex without coating or powder. | | |
| 3. Natural rubber latex without coating but powdered. | | |
| 4. Natural rubber latex with polyurethane polymer coating but without polyethylene wax, prepared essentially as described in Example 2. | | |
| 5. Natural rubber latex with polyurethane polymer coating but without polyethylene wax, prepared essentially as described in Example 4. | | |

It will be understood that the invention disclosed and defined in this specification extends to all alternative combinations of two or more of the individual features mentioned or evident from the text or drawings. All of these different combinations constitute various alternative aspects of the invention.

It will also be understood that the term "comprises" (or its grammatical variants) as used in this specification is equivalent to the term "includes" and should not be taken as excluding the presence of other elements or features.

## Claims

1. An elastomeric article having a layer of natural or synthetic rubber to which is applied at least one layer of a coating of a polymer blend **characterised in that** the polymer blend includes at least one film forming polymer and at least one wax.

2. An article according to claim 1 wherein the first layer of natural or synthetic rubber is selected from natural rubber latex, nitrile rubber latex, polychloroprene, styrene-butadiene rubber or a polyurethane.

3. An article according to claim 1 or claim 2 wherein the film forming polymer is selected from acrylic polymers, methacrylate polymers, polyurethane polymers, carboxylated styrene-butadiene polymers and copolymers thereof.

4. An article according to any one of claims 1 to 3 inclusive wherein the wax is selected from synthetic polymer and copolymer waxes.

5. An article according to any one of claims 1 to 4 inclusive wherein the natural or synthetic rubber includes a natural rubber latex or polychloroprene.

6. An article according to any one of claims 1 to 5 inclusive wherein the film forming polymer includes a polyurethane.

7. An article according to any one of claims 1 to 6 inclusive wherein the wax is a synthetic polymer or copolymer wax selected from polyethylene wax, high density polyethylene wax, an oxidised or modified polyethylene wax or high density polyethylene wax or a mixture of said waxes.

8. An article according to any one of claims 1 to 7 inclusive wherein the wax is a polyethylene wax.

9. An article according to any one of claims 1 to 8 inclusive wherein the wax is a polyethylene wax having a melting point greater than or equal to 80°C.

10. An artide according to any one of claims 1 to 9 inclusive wherein the polymer blend further includes a hardness modifier.

11. An article according to claim 10 wherein the hardness modifier has a glass transition temperature higher than that of the film forming polymer.

12. An article according to claim 10 or claim 11 wherein the hardness modifier is selected from a melamine formaldehyde resin, an acrylic polymer, a poly(2-hydroxythethyl methacrylate) or a mixture thereof.

13. An article according to any one of claims 1 to 12 inclusive wherein said article is a surgeons' glove and wherein the first layer comprises the outer layer and the coating of a polymer blend comprises the inner layer.

14. An article according to claim 13, wherein the first layer is 0.10-0.40 mm thick and the coating is 1-10 µm thick.

15. An article according to any one of claims 1 to 14 inclusive wherein said article has been further treated with a silicone emulsion.

16. An article according to claim 15, wherein the silicone emulsion further includes a surfactant.

17. A process for making an elastomeric article which process includes forming a layer of natural or synthetic rubber followed by coating at least one surface of the layer of natural or synthetic rubber with a layer of a polymer blend including at least one film forming polymer and a wax.

18. A process for making an elastomeric article as defined according to any one of claims 1 to 16 inclusive which process includes the following steps:
(a) dipping a suitable former in a coagulant;
(b) forming a rubber article by dipping the coagulant coated former in a rubber latex;
(c) optionally gelling the rubber article by heating;
(d) optionally leaching the rubber article in hot water;
(e) optionally priming the rubber-article by treating with a dilute aqueous solution of a hypochlorite or an acid;
(f) optionally leaching the rubber article in hot water;
(g) optionally dipping the rubber article in a dilute solution of aluminium sulphate or calcium nitrate;
(h) dipping the rubber article in a solution or dispersion of a polymer blend including a film forming polymer and a wax to form a coating of the polymer blend on the rubber article;
(i) heat drying the resulting coating in order to bond the polymer blend to the rubber layer;
(j) optionally leaching the elastomeric article in hot water;
(k) removing the elastomeric article from the former;
(l) optionally leaching the elastomeric article in hot water;
(m) optionally treating the article with an aqueous dispersion or solution of a silicone and a surfactant; and
(n) drying the elastomeric article.

19. A process according to claim 17 or claim 18 wherein the first or rubber layer includes a natural rubber latex and the polymer blend includes a polyurethane film forming polymer and a polyethylene wax.

## Revendications

1. Article élastomère ayant une couche de caoutchouc naturel ou synthétique, sur laquelle on applique au moins une couche d'un revêtement d'un mélange de polymères, **caractérisé en ce que** le mélange de polymères inclut au moins un polymère formant un film et au moins une cire.

2. Article selon la revendication 1, dans lequel la première couche de caoutchouc naturel ou synthétique est choisie parmi latex de caoutchouc naturel, latex de caoutchouc nitrile, polychloroprène, caoutchouc styrène-butadiène ou un polyuréthanne.

3. Article selon la revendication 1 ou 2, dans lequel le polymère formant un film est choisi parmi polymères acryliques, polymères de méthacrylate, polymères de polyuréthanne, polymères de styrène-butadiène carboxylés et copolymères de ceux-ci.

4. Article selon l'une quelconque des revendications 1 à 3 incluse, dans lequel la cire est sélectionnée parmi cires de copolymère et polymère synthétique.

5. Article selon l'une quelconque des revendications 1 à 4 incluse, dans lequel le caoutchouc naturel ou synthétique inclut un latex de caoutchouc naturel ou un polychloroprène.

6. Article selon l'une quelconque des revendications 1 à 5 incluse, dans lequel le polymère formant un film inclut un polyuréthanne.

7. Article selon l'une quelconque des revendications 1 à 6 incluse, dans lequel la cire est une cire de copolymère ou polymère synthétique choisie parmi cire de polyéthylène, cire de polyéthylène haute densité, une cire de polyéthylène oxydée ou modifiée, une cire de polyéthylène haute densité, ou un mélange desdites cires.

8. Article selon l'une quelconque des revendications 1 à 7 incluse, dans lequel la cire est une cire de polyéthylène.

9. Article selon l'une quelconque des revendications 1 à 8 incluse, dans lequel la cire est une cire de polyéthylène ayant un point de fusion supérieur ou égal à 80° C.

10. Article selon l'une quelconque des revendications 1 à 9 incluse, dans lequel le mélange de polymères inclut en outre un modificateur de dureté.

11. Article selon la revendication 10, dans lequel le modificateur de dureté a une température de transition vitreuse supérieure à celle du polymère formant un film.

12. Article selon la revendication 10 ou 11, dans lequel le modificateur de dureté est choisi parmi une résine de mélanine-formaldéhyde, un polymère acrylique, un poly(2-hydroxythéthyl méthacrylate) ou un mélange de ceux-ci.

13. Article selon l'une quelconque des revendications 1 à 12 incluse, dans lequel ledit article est un gant de chirurgie, et dans lequel la première couche comprend la couche externe et le revêtement d'un mélange polymère comprend la couche interne.

14. Article selon la revendication 13, dans lequel la première couche a une épaisseur de 0,10 à 0,40 mm, et le revêtement a une épaisseur de 1 à 10 µm.

15. Article selon l'une quelconque des revendications 1 à 14 incluse, dans lequel ledit article a été traité, en outre, avec une émulsion de silicone.

16. Article selon la revendication 15, dans lequel l'émulsion de silicone inclut, en outre, un tensioactif.

17. Procédé de fabrication d'un article élastomère, lequel procédé inclut la formation d'une couche de caoutchouc naturel ou synthétique, suivie par le revêtement d'au moins une surface de la couche de caoutchouc naturel ou synthétique avec une couche d'un mélange de polymères incluant au moins un polymère formant un film et une cire.

18. Procédé de fabrication d'un article élastomère selon l'une quelconque des revendications 1 à 16 incluse, lequel procédé inclut les étapes suivantes consistant à :
(a) plonger une forme appropriée dans un coagulant ;
(b) former un article en caoutchouc en plongeant la forme revêtue du coagulant dans un latex de caoutchouc ;
(c) gélifier, de façon facultative, l'article en caoutchouc par chauffage ;
(d) lessiver, de façon facultative, l'article en caoutchouc dans de l'eau chaude ;
(e) amorcer, de façon facultative, l'article en caoutchouc en le traitant avec une solution aqueuse diluée d'hypochlorite ou un acide ;
(f) lessiver, de façon facultative, l'article en caoutchouc dans de l'eau chaude ;
(g) plonger, de façon facultative, l'article en caoutchouc dans une solution diluée de sulfate d'aluminium ou de nitrate de calcium ;
(h) plonger l'article en caoutchouc dans une solution ou une dispersion d'un mélange de polymères incluant un polymère formant un film et une cire, pour former un revêtement du mélange de polymères sur l'article en caoutchouc ;
(i) sécher à la chaleur le revêtement ainsi obtenu afin de lier le mélange de polymères à la couche de caoutchouc ;
(j) lessiver, de façon facultative, l'article élastomère dans de l'eau chaude ;
(k) retirer l'article élastomère de la forme ;
(l) lessiver, de façon facultative, l'article élastomère dans de l'eau chaude ;
(m) traiter, de façon facultative, l'article avec une dispersion ou une solution aqueuse d'une silicone et d'un tensioactif ; et
(n) sécher l'article élastomère.

19. Procédé selon la revendication 17 ou 18, dans lequel la première couche ou la couche en caoutchouc inclut un latex de caoutchouc naturel, et le mélange de polymères inclut un polymère formant un film de polyuréthanne et une cire de polyéthylène.

## Patentansprüche

1. Elastomerer Gegenstand mit einer Schicht aus natürlichem oder synthetischem Kautschuk, auf die mindestens eine Schicht einer Beschichtung aus einer Polymermischung aufgetragen wird, **dadurch gekennzeichnet, daß** die Polymermischung mindestens ein Film-bildendes Polymer und mindestens ein Wachs umfaßt.

2. Gegenstand nach Anspruch 1, wobei die erste Schicht aus natürlichem oder synthetischem Kautschuk aus natürlichem Kautschuklatex, Nitril-Kautschuklatex, Polychloropren, StyrolButadien-Kautschuk oder einem Polyurethan ausgewählt ist.

3. Gegenstand nach Anspruch 1 oder Anspruch 2, wobei das Film-bildende Polymer aus Acrylpolymeren, Methacrylatpolymeren, Polyurethanpolymeren, carboxylierten Styrol-Butadienpolymeren und Copolymeren davon ausgewählt ist.

4. Gegenstand nach einem der Ansprüche 1 bis einschließlich 3, wobei das Wachs aus synthetischen Polymer- und Copolymerwachsen ausgewählt ist.

5. Gegenstand nach einem der Ansprüche 1 bis einschließlich 4, wobei der natürliche oder synthetische Kautschuk einen natürlichen Kautschuklatex oder Polychloropren umfaßt.

6. Gegenstand nach einem der Ansprüche 1 bis einschließlich 5, wobei das Film-bildende Polymer ein Polyurethan umfaßt.

7. Gegenstand nach einem der Ansprüche 1 bis einschließlich 6, wobei das Wachs ein synthetisches Polymer- oder Copolymerwachs ist, ausgewählt aus Polyethylenwachs, Wachs aus Polyethylen hoher Dichte, einem oxidierten oder modifizierten Polyethylenwachs oder einem Wachs aus Polyethylen hoher Dichte oder einem Gemisch aus diesen Wachsen.

8. Gegenstand nach einem der Ansprüche 1 bis einschließlich 7, wobei das Wachs ein Polyethylenwachs ist.

9. Gegenstand nach einem der Ansprüche 1 bis einschließlich 8, wobei das Wachs Polyethylenwachs mit einem Schmelzpunkt größer als oder gleich 80 °C ist.

10. Gegenstand nach einem der Ansprüche 1 bis einschließlich 9, wobei die Polymermischung ferner einen Härtemodifizierer umfaßt.

11. Gegenstand nach Anspruch 10, wobei der Härtemodifizierer eine Glasübergangstemperatur höher als die des Film-bildenden Polymers hat.

12. Gegenstand nach Anspruch 10 oder Anspruch 11, wobei der Härtemodifizierer aus einem Melaminformaldehydharz, einem Acrylpolymer, einem Poly(2-hydroxyethylmethacrylat) oder einem Gemisch davon ausgewählt ist.

13. Gegenstand nach einem der Ansprüche 1 bis einschließlich 12, wobei der Gegenstand ein chirurgischer Handschuh ist und wobei die erste Schicht die äußere Schicht umfaßt und die Beschichtung aus einer Polymermischung die innere Schicht umfaßt.

14. Gegenstand nach Anspruch 13, wobei die erste Schicht 0,10 - 0,40 mm dick ist und die Beschichtung 1 - 10 µm dick ist.

15. Gegenstand nach einem der Ansprüche 1 bis einschließlich 14, wobei der Gegenstand weiter mit einer Silikonemulsion behandelt wird.

16. Gegenstand nach Anspruch 15, wobei die Silikonemulsion ferner ein oberflächenaktives Mittel umfaßt.

17. Verfahren zur Herstellung eines elastomeren Gegenstandes, wobei das Verfahren die Bildung einer Schicht aus natürlichem oder synthetischem Kautschuk, gefolgt von der Beschichtung mindestens einer Oberfläche der Schicht aus natürlichem oder synthetischem Kautschuk mit einer Schicht aus einer Polymermischung, die ein Film-bildendes Polymer und ein Wachs umfaßt, umfaßt.

18. Verfahren zur Herstellung eines elastomeren Gegenstandes wie nach einem der Ansprüche 1 bis einschließlich 16 definiert, wobei das Verfahren die folgenden Schritte umfaßt:
(a) Eintauchen einer geeigneten Tauchform in ein Koagulationsmittel;
(b) Bilden eines Kautschukgegenstandes durch das Eintauchen der Koagulationsmittel beschichteten Tauchform in einen Kautschuklatex;
(c) gegebenenfalls Gelierung des Kautschukgegenstandes durch Erwärmung;
(d) gegebenenfalls Auslaugung des Kautschukgegenstandes in heißem Wasser;
(e) gegebenenfalls Grundieren des Kautschukgegenstandes durch Behandlung mit einer verdünnten wässerigen Lösung aus Hypochlorit oder einer Säure;
(f) gegebenenfalls Auslaugung des Kautschukgegenstandes in heißem Wasser;
(g) gegebenenfalls Eintauchen des Kautschukgegenstandes in eine verdünnte Lösung aus Aluminiumsulfat oder Calciumnitrat;
(h) Eintauchen des Kautschukgegenstandes in eine Lösung oder Dispersion aus einer Polymermischung, die ein Film-bildendes Polymer und ein Wachs umfaßt, um eine Beschichtung aus einer Polymermischung auf dem Kautschukgegenstand zu bilden;
(i) Wärmetrocknen der resultierenden Beschichtung, um die Polymermischung an die Kautschukschicht zu binden;
(j) gegebenenfalls Auslaugen des elastomeren Gegenstandes in heißem Wasser;
(k) Entfernen des elastomeren Gegenstandes aus der Tauchform;
(l) gegebenenfalls Auslaugen des elastomeren Gegenstandes in heißem Wasser;
(m) gegebenenfalls Behandeln des Gegenstandes mit einer wässerigen Dispersion oder Lösung aus einem Silikon und einem oberflächenaktiven Mittel; und
(n) Trocknen des elastomeren Gegenstandes.

19. Verfahren nach Anspruch 17 oder Anspruch 18, wobei die erste oder die Kautschukschicht einen natürlichen Kautschuklatex umfaßt, und die Polymermischung ein Polyurethanfilm-bildendes Polymer und ein Polyethylenwachs umfaßt.
